# EUROPÄISCHE PATENTSCHRIFT

(11) **EP 2 564 778 B1**
(45) Veröffentlichungstag und Bekanntmachung des Hinweises auf die Patenterteilung: **25.04.2018**
(21) Anmeldenummer: 12006141.1
(22) Anmeldetag: 29.08.2012
(51) Int. Cl.: A61B 5/20

(54) **Vorrichtung und Verfahren zur Harnflussmessung**
Device and method for urine flow measurement
Dispositif et procédé destiné à mesurer le débit urinaire

(30) Priorität: 29.08.2011 DE 102011111982
(43) Veröffentlichungstag der Anmeldung: 06.03.2013
(73) Patentinhaber: Wiest Uropower Ltd., 14478 Potsdam (DE)
(72) Erfinder: Wiest, Friedhelm, Michael, 14199 Berlin (DE)
(74) Vertreter: Fritzsche, Thomas

(56) Entgegenhaltungen:
- DE-A1- 2 623 557
- DE-C2- 3 007 855
- US-A- 4 149 411
- US-A1- 2004 211 267

## Beschreibung

Die Erfindung betrifft eine Vorrichtung zur Messung einer Harnströmung eines Patienten mit einem Messbecher, der gegenüber einem Messwertgeber angeordnet ist. Die Erfindung betrifft ferner ein Verfahren zur Messung einer Harnströmung eines Patienten, bei dem der Messbecher gegenüber dem Messwertgeber angeordnet wird.

Die Analyse des Harnflusses ist eine bekannte Methode zur Diagnose urologischer Störungen. Messgeräte, die zu diesem Zwecke Verwendung finden, werden zumeist als Uroflow, Urometer, Uroflowmeter oder einfach Flowmeter bezeichnet. Durch die Analyse des Strömungsverlaufes über die Zeit ist es beispielsweise möglich, Behinderungen im unteren Urogenitaltrakt festzustellen oder aber Blasenentleerungsstörungen zu diagnostizieren. Typische Merkmale, die zur Diagnostik herangezogen werden, sind die Gesamtdauer des Harnlassens (dieser Vorgang wird auch als Miktion bezeichnet), maximaler Fluss, mittlerer Fluss, Gesamtvolumen und Kurvenverlauf des Flussprofiles, wobei das Volumen über die Zeit aufgetragen wird. Durch Vergleich der Messdaten mit Mittelwerten gesunder bzw. erkrankter Patientenkollektive lässt sich beispielsweise der Schweregrad einer urethralen Striktur ableiten. Die Analyse des Harnflusses, die sog. Uroflowmetrie ist ferner sinnvoll in der Diagnose einer etwaigen Prostatavergrößerung und wird darüber hinaus postoperativ zur Prüfung des Therapie-/ Operationserfolges eingesetzt.

Es gibt eine Vielzahl von Vorrichtungen und Methoden, welche den Stand der Technik zur Messung des Harnflusses darstellen.

In DE 25 50 906 A1 ist eine Vorrichtung zur Messung der maximalen Urinmenge je Miktion beschrieben, wobei eine einfache Sensorik hierfür in Form eines Markierungsstreifens realisiert ist, welcher den höchsten Wasserstand in einem Messrohr markiert.

In DE 22 59 612 C3 wird eine Vorrichtung zur Messung der maximalen Harnmenge beschrieben, welche sich dadurch auszeichnet, dass sie sehr kostengünstig herstellbar ist. Die Vorrichtung ist aus verschweißten Beuteln aufgebaut, wobei die Schweißnähte einen oberen trichterförmigen Teil mit aufgedruckter Messskala ausbilden, sowie einen unteren Teil, der als Auffangbehälter dient. Oberer und unterer Teil sind mittels Klemme (z.B. durch eine Gefäßklemme) verschließbar. Wie die in DE 25 50 906 A1 beschriebene Vorrichtung ist auch diese Vorrichtung nicht dazu in der Lage, außer der Maximalmenge andere Größen messtechnisch zu bestimmen. Allerdings ist die Einfachheit und Kostengünstigkeit dieser hygienisch einwandfreien Lösung erwähnenswert.

In DE 19 61 33 06 C2 sowie in DE 29 50 65 00 U1 sind Aufbauten beschrieben, in denen jeweils ein Harnsammelbehälter mit einem Auffangbehälter durch eine automatisiert betriebene Quetschvorrichtung am untersten Punkt verschlossen wird. Der gesamte Aufbau ist in eine Waage eingespannt und über einer Toilette oder einem Abfluss positioniert. Reizvoll an dieser Lösung ist die zu erwartende hohe Messgenauigkeit durch das Wägeprinzip (wobei Impulsanteile der Flüssigkeitsströmung als Messfehler in Erscheinung treten können) bei gleichzeitiger Lösung der typischen Handhabungsproblematik des Wägeprinzips, nämlich der Notwendigkeit einer manuellen Entleerung oder Entsorgung und/ oder Reinigung eines Auffangbehälters durch Bedienpersonal.

In GB 24 37 549 A wird eine robuste Lösung vorgeschlagen, bei der eine Wartung nebst manueller Entleerung eines Auffanggefäßes weitestgehend entfällt. Die Messeinrichtung ist hier in die Toilette integriert, wobei der Toilettensitz ein Bestandteil des Apparates ist. Das Messprinzip ist auch hier jenes der Wägemessung. Ein Auffangbehälter wird über eine mit einem (oder mehreren) Dehnungsmessstreifen versehenen Haltevorrichtung mittig in der Toilette gehalten. Nach Ende der Miktion kann nun manuell oder automatisiert am Boden des Auffangbehälters ein Ausfluss geöffnet werden. Ferner wird die Toilettenspülung zur Reinigung des Messgerätes eingesetzt.

Ein ähnlicher Aufbau ist in der US 45 54 687 A beschrieben. Auch in dieser Druckschrift wird ein Auffanggefäß zum Einsetzen in eine Toilette beschrieben, wobei dieses im Gegensatz zu GB 24 37 549 A als Einmalprodukt ausgeführt wird. Das Gefäß weist am unteren Becherrand einen Schlauchanschluss auf, welcher mit einem wieder verwendbaren Drucksensor verbunden ist. Das Messprinzip ist die Auswertung des statischen Druck einer sich durch eine ansteigende Flüssigkeit komprimierenden Luftsäule.

In DE 26 235 57 A1 ist eine rohrförmige Vorrichtung beschrieben, in welcher der aufgefangene Urin gesammelt wird und durch übereinander liegende Bohrungen in der Wandung des Rohres in ein Auffanggefäß austritt. Je stärker der Urinfluss ist, desto höher wird die Flüssigkeitssäule sich im Rohr aufstauen. Die Auswertung erfolgt beispielsweise optisch. Nachteilig an diesem Aufbau sind der hohe Wartungsaufwand, da der aufgefangene Urin manuell entleert werden muss, sowie die unbefriedigende Möglichkeit zur Reinigung. Ferner ist mit Nichtlinearitäten beim Ausströmen durch die Bohrungen zu rechnen, weshalb ein genaues Messen des Flussprofils nicht möglich ist.

In der DE 39 33 025 C2 wird ebenfalls eine Messeinrichtung vorgeschlagen, welche über das Prinzip des Staudrucks den Volumenstrom bestimmt. Die Messdatenerfassung erfolgt hier mit einem Druckaufnehmer, welcher die Höhe der sich stauenden Wassersäule erfasst. Vorteilhafterweise kann das Gerät ohne einen Auffangbehälter betrieben werden, was die Wartungsfreiheit unterstützt. Das Messgerät wird hier direkt über einer Toilettenschüssel positioniert. Alternativ bzw. in Ergänzung kann das System jedoch auch mit Auffangbehälter betrieben werden, was der Erhöhung der Messgenauigkeit und zu Kalibrierungszwecken dienlich ist.

Die Offenlegungsschrift DE 41 09 974 A1 nimmt Bezug auf die DE 39 33 025 C2 und schlägt eine Methodik vor, um Messfehler aufgrund von Oberflächenspannungseffekten zu minimieren bzw. zu eliminieren.

In der Offenlegungsschrift DE 35 41 649 A1 ist wiederum eine Vorrichtung beschrieben, bei der der Harnstrom auf eine rotierende Scheibe axial geleitet wird und diese radial verlässt. Das an der Antriebswelle entstehende Drehmoment (Bremsmoment) ist proportional zum Fluss, so dass die Messung des Flusses über die Stromaufnahme des Motors möglich ist. Obgleich dieser Aufbau im Grundsatz eine kompakte Bauform zulässt, ist die Lösung vermutlich nicht hinreichend messgenau, insbesondere zu Beginn und zum Ende einer Miktion. Die Reinigung eines solchen Systems dürfte je nach Ausführungsform problematisch sein.

Die technische Lehre der DE 34 90 149 T5 zeigt Ähnlichkeiten zu derjenigen der DE 35 41 649, da auch in dem letztgenannten Dokument eine sich schnell drehende Einrichtung zum Einsatz kommt. Die auftreffende Flüssigkeit wird hier durch die sich drehende Scheibe radial abgelenkt, sodass die Flüssigkeit in etwa rotationssymmetrisch verteilt auf die umliegende Becherwandung trifft. Die Flüssigkeit, der die Impulsanteile des Harnstrahls genommen sind, läuft nun entlang der Becherwandung in ein Sammelgefäß. Die eigentliche Auswertung erfolgt nach dem Prinzip der Wägemessung.

In der DE 300 78 55 C2 wird als Vorrichtung zur Flussmessung ein Messwertgeber vorgeschlagen, bei dem ein schnecken- (oder allgemein U- förmig) ausgebildetes Durchflusselement dazu dient, einen Teil des Urins eine hinreichende Zeit beim Durchströmen im Messwertgeber zu belassen und das Gewicht je Zeiteinheit zu messen. Um eine Verfälschung der Messung durch die vorhandenen Impulsanteile weitestgehend zu verhindern, ist hier am Einlauf eine Prallplatte vorgesehen, welche die kinetische Energie des Flusses aufnehmen soll. Vorteilhaft an dieser Vorrichtung ist die Möglichkeit des Verzichts auf einen Auffangbehälter, so dass ein Einsetzen in ein Toilettenbecken möglich ist und die manuelle Reinigung entfällt. Nachteilig an diesem Aufbau ist die Notwendigkeit eines sehr genauen horizontalen Ausrichtens des Sensors, da die Lage des Durchflusselementes einen großen Einfluss auf das Durchströmen des Durchflusselementes und damit auf die Messcharakteristik hat. Ferner ist das Einsetzen in ein Urinal in der vorgestellten Bauform nicht möglich.

In DE 198 22 988 C2 ist hingegen ein Messwertgeber beschrieben, welcher in einem handelsüblichen Urinal eingesetzt werden kann. Die Flüssigkeitsströmung trifft hier auf ein Prallelement, welches an einem verformbaren einseitig eingespannten Hebelarm angebracht ist. Durch das Auftreffen auf die Prallplatte wird ein zeitlich sich mit dem Fluss verändernder Impuls übertragen, welcher den Hebelarm verbiegt. Die Verbiegung wird durch einen Dehnungsmessstreifen erfasst. Nachteilig an dieser Lösung ist, dass die Gewichtskraft der Flüssigkeit auf dem Prallelement als Messfehler eingeht.

Ein ähnlicher Aufbau ist in US 50 78 012 A beschrieben. Auch hier erfolgt eine Impulsmessung, indem ein Flüssigkeitsstrahl mit bestimmter Geschwindigkeit auf einen elastischen Tragarm trifft, welcher von der Strömung ausgelenkt wird. Die gemessene Auslenkung ist analog DE 198 22 988 C2 proportional zum Fluss. Auch hier ist mit einer Überlagerung aus nicht vollständig zu vermeidender Wägemessung als Störeffekt und der gewünschten Impulsmessung zu rechnen.

Ein vergleichsweise exotisches Messprinzip wird in der DE 69 92 52 88 T2 vorgeschlagen: Der Messwertgeber ist hier in einen einmal verwendbaren Sammelbehälter und einen mehrfach verwendbaren Mess- und Auswertungsteil unterteilt. Mit Hilfe eines Lautsprechers im mehrfach verwendbaren Teil wird die Luftsäule zwischen Lautsprecher und Flüssigkeitsspiegel im einmal verwendbaren Teil in Resonanz versetzt. Durch Veränderung des Füllstandes aufgrund des Zuflusses von Urin variiert das Luftvolumen, was eine Frequenzveränderung zur Folge hat und mit einem Mikrophon im mehrfach verwendbaren Teil detektiert wird. Insgesamt erscheint der vorgeschlagene Aufbau sehr wartungsintensiv und eher für einen stationären Klinikaufenthalt geeignet als für die Routine in einer urologischen Praxis.

Auch in der US 2005 02 56 428 A1 wird ein unübliches Messprinzip beschrieben. Urin wird hier in/mit einem Trichter gesammelt und durch ein magnetisches Feld (konkret zwischen zwei Magneten) durchgeleitet. Beim Durchfließen wird eine flowabhängige Spannung induziert gemäß dem faradaysches Gesetz, nachdem eine Spannung in eine Leitung induziert wird, wenn diese senkrecht durch ein Magnetfeld bewegt wird. Da die Spannung proportional zur Geschwindigkeit des Leiters ist, kann hiermit die Fließgeschwindigkeit und bei bekanntem Querschnitt der Volumenstrom ermittelt werden. Enorm vorteilhaft an dem Aufbau ist die Tatsache, dass keine bewegten Teile verwendet werden. Es ist folglich nicht mit einem mechanischen Verschleiß und damit einer etwaigen Änderung der Messcharakteristik zu rechnen, was eine geringe Wartungsnotwendigkeit verspricht. Da die gemessene Spannung von der Leitfähigkeit des "Leiters" (also des Urins) abhängig ist, diese aber nicht als konstant angenommen werden kann aufgrund variabler Bestandteile und somit auch Ionenzusammensetzung, ist mit einem gewissen Messfehler zu rechnen.

In DE 19 70 51 111 C2 ist eine Vorrichtung beschrieben, die im wesentlichen eine Prallplatte aufweist, welche einseitig drehbeweglich gelagert ist und die Winkelauslenkung dieser Platte durch das Auftreffen einer Harnströmung durch ein Sensorelement erfasst wird. Die Prallplatte ist hier insbesondere dadurch gekennzeichnet, dass ein Magnet, vorzugsweise ein Ringmagnet, an dieser Platte befestigt und dieser "bewegliche" Magnet zu einem an dem Messaufbau starr befestigten Stabmagneten assoziiert ist, wobei die Polung der Magnete so gestaltet ist, dass diese sich in abstoßender Richtung gegenüberstehen. Aufgrund der magnetischen Wechselwirkung zwischen beiden Magneten wird die Auslenkung der Prallplatte in einer Ruhelage gehalten. Das Auftreffen des Harns auf die Prallplatte führt zu einer Auslenkung aus dieser Ruhelage. Eine automatische Nullpunktkalibrierung soll zeitabhängige Veränderungen kompensieren. Nachteilig an dieser Vorrichtung sind der komplexe Aufbau und eine nicht unbeschränkte Integrierbarkeit in ein vorhandenes Urinal.

Allen Systemen ist gemein, dass Ihr Einsatz ausschließlich Kliniken und speziellen urologischen Arztpraxen vorbehalten ist. Nur hier ist die nach jedem Messvorgang notwendige Reinigung von Rückständen sichergestellt, Austausch etwaig notwendigen Verbrauchsmaterials, sowie die Pflege der teils sehr empfindlichen Sensorik.

Ein Einsatz in einem nichtmedizinischen Umfeld, zum Beispiel in öffentlichen (Herren-) Toiletten, zum Zwecke eines flächendeckenden Screenings ist mit den im Stand der Technik beschriebenen Vorrichtungen nicht möglich, da Urinstein, Zigarettenkippen oder sonstiger Unrat sich im Sensor ablagern, die Kalibrierung verfälschen oder gar den Messwertgeber zerstören könnten.

Unter Screening ist hierbei die Untersuchung vermeintlich gesunder Bevölkerungsgruppen zu verstehen zum Zwecke der Früherkennung erster Anzeichen einer möglichen Erkrankung, wie beispielsweise eine Prostatavergrößerung, welche auf ein sich manifestierendes Prostatakarzinom hinweisen kann.

Insbesondere für Männer, welche im Gegensatz zu Frauen, die regelmäßig den Gynäkologen aufsuchen, ohne Anzeichen einer Erkrankung oder sonstiger Beschwerden typischerweise nicht, vor allem nicht regelmäßig, einen Andrologen oder Urologen aufsuchen, wäre die Verfügbarkeit einer einfachen, verlässlichen, diskreten und selbstbestimmten Uroflow- Untersuchung vorteilhaft, um hierdurch etwaige Erkrankungen frühzeitig feststellen und die Empfehlung aussprechen zu können, eine urologische Facharztpraxis zwecks umfassender weiterer Diagnostik aufzusuchen.

Durch eine somit verbesserte Früherkennung könnten einerseits spätere volkswirtschaftlich relevante hohe Kosten z.B. durch onkologische Therapie und Arbeitsausfall im Vorfeld vermieden oder wenigstens stark begrenzt werden, gleichzeitig könnten hohe Kosten für das Gesundheitswesen durch die alternativ regelmäßig erforderlichen Facharztbesuche vermieden werden, da dieser erst nach einem positiven Befund aus der Eigenmessung aufgesucht werden würde.

Während eine Robustheit und Wartungsfreiheit der Vorrichtung zur Harnflussmessung, sowie die Selbstbedienbarkeit dieser Vorrichtung durch den Anwender Grundbedingungen sind, um in einem nichtmedizinischen Umfeld überhaupt erfolgreich und verlässlich Messungen vornehmen zu können, sind diese Aspekte auch im klassischen medizinischen Umfeld von hoher Relevanz, da aufgrund des zunehmenden Kostendruckes im Gesundheitswesen Lösungen, welche einen intensiven Personaleinsatz zur Reinigung, Bedienung, Entleerung von Sammelbehältern und Auswertung der Messungen erforderlich machen, aus wirtschaftlichen Gesichtspunkten schlichtweg inakzeptabel sind. Ferner ist das urinieren in ein Messgerät unter Beisein eines Bedieners unangenehm für den Patienten. Obendrein zeigen Untersuchungen, dass das Harnverhalten unter Beobachtung anders ist als unter entspannten und selbstbestimmten Bedingungen, bei denen die Privatsphäre gewahrt ist. Die medizinische Aussage einer Uroflowmessung kann sich folglich allein durch die Anwesenheit eins Arztes/ einer Schwester verschlechtern oder verfälschen.

In US4149411 ist ein Milchproduktionsmeter beschrieben, bei dem ein Fluid, wie beispielsweise Milch, durch einen Einlassdurchgang in das Meter eingeleitet und in einem Fluidbehälter von mindestens einem Teil empfangen wird, der beweglich ist, wobei mindestens ein Teil der Bewegung in Reaktion auf den Empfang des Fluids erfolgt, wobei das Fluid dann von dem Meter durch einen Auslasskanal ausgestoßen wird. Ein Sensor erfasst die Bewegung des beweglichen Teils, die verursacht ist durch den Empfang von Fluid in dem Fluidbehälter, und, in Reaktion darauf, liefert ein elektrisches Signal, das die Menge der Flüssigkeit anzeigt.

Zusammenfassend lässt sich feststellen, dass die im Stand der Technik vorbeschriebenen Vorrichtungen lediglich funktionalen Aspekten der Messung einigermaßen genügen. Andere technische Aspekte, so beispielsweise die Integrierbarkeit eines Messwertgebers in ein vorhandenes Urinal, sind aus Sicht urologischer Facharztpraxen nicht befriedigend gelöst.

Ferner ist der Einsatz der im Stand der Technik beschriebenen Vorrichtungen auch aus Kostengesichtspunkten hinsichtlich der eingesetzten Mess- und Auswertungseinrichtungen für einen flächendeckenden Einsatz, insbesondere im Rahmen eines Screening, ungeeignet.

Der Erfindung liegt daher die Aufgabe zu Grunde, die Nachteile der beschriebenen bekannten Vorrichtungen dadurch zu vermeiden, dass eine einfache, weitestgehend wartungsfreie und gegen Vandalismus schützbare und folglich robuste, kostengünstige, zudem in bestehende Sanitäranlagen, insbesondere Urinale, nachrüstbare und selbstbedienbare Vorrichtung zur Messung der Harnströmung und ein entsprechendes Verfahren zur Messung der Harnströmung sowohl für das medizinisehe wie auch nichtmedizinische Umfeld zur Verfügung gestellt werden.

Die erfindungsgemäße Vorrichtung zur Messung einer Harnströmung eines Patienten umfasst einen Messbecher, der gegenüber einem Messwertgeber derart angeordnet ist, dass eine Verschiebung des Messbechers gegenüber dem Messwertgeber als Maß für ein Volumen von in dem Messbecher gesammeltem Urin detektierbar ist, wobei die Verschiebung von einer Erhöhung eines Gesamtgewichtes des Messbechers bewirkt ist, die beim Strömen des Urins durch den Messbecher hindurch dem Gewicht des im Messbecher gesammelten Urins entspricht. Im Folgenden werden Urin und Harn als zueinander synonyme Begriffe verwendet. Unter einer Verschiebung wird insbesondere ein Lageunterschied des Messbechers gegenüber dem Messwertgeber in Richtung der Schwerkraft, die auf den Urin wirkt, verstanden, der entsteht, wenn der Messbecher von dem Urin durchströmt oder durchflossen wird. Die Verschiebung kann als eine translatorische Bewegung, eine rotatorische Bewegung, oder als Kombination aus einer translatorischen und rotatorischen Bewegung des Messbechers gegenüber dem Messwertgeber erfolgen. Die Verschiebung kann im Messwertgeber und/oder im Messbecher detektiert werden. Der Messwertgeber erzeugt aus dem detektierten Signal einen Messwert, der an eine Auswertungseinheit übertragbar ist. Der Messbecher kann in oder an dem Messwertgeber gelagert angeordnet sein. Die Lagerung des Messbechers kann mittels eines Federelementes, das zwischen dem Messbecher und dem Messwertgeber angeordnet ist, oder berührungslos mittels an dem Messbecher und dem Messwertgeber angeordneten Magneten ausgeführt sein. Die Verschiebung kann besonders einfach detektiert werden, wenn ohne ein Strömen des Urins durch den Messbecher hindurch eine Ruhelage oder Ruheposition des Messbechers gegenüber dem Messwertgeber definiert ist, in der eine auf den Messbecher wirkende Gewichtskraft durch eine dieser Gewichtskraft entgegengesetzte weitere Kraft, beispielsweise eine Federkraft und/oder eine Magnetkraft, ausgeglichen ist. Bei einer Verschiebung des Messbechers gegenüber dem Messwertgeber wirkt die weitere Kraft, die der auf den Messbecher wirkenden Gewichtskraft entgegengesetzt ist, als Rückstellkraft in die Ruhelage oder Ruheposition. Mit der erfindungsgemäßen einfachen Anordnung aus Messwertgeber und dem Messwertgeber gegenüber verschiebbarem Messbecher wird eine einfache, weitestgehend wartungsfreie und gegen Vandalismus schützbare und folglich robuste, kostengünstige, zudem in bestehende Sanitäranlagen, insbesondere Urinale, nachrüstbare und selbstbedienbare Vorrichtung zur Messung der Harnströmung bereitgestellt. An dem Messbecher ist zur Lagerung des Messbechers gegenüber dem Messwertgeber ein Ringmagnet oder eine Mehrzahl von Magneten gleicher Polungsrichtung angebracht, wobei an dem Messwertgeber ein zweiter Ringmagnet oder eine Mehrzahl von zweiten Magneten gleicher Polungsrichtung angebracht ist. Die Magnetpole des Ringmagneten oder der Mehrzahl von Magneten sind zu den Magnetpolen des zweiten Ringmagneten oder der Mehrzahl von zweiten Magneten angeordnet, um eine abstoßende oder anziehende Magnetkraft zwischen dem Messbecher und dem Messwertgeber zu erzeugen. Die Magnetkraft wirkt derart, dass eine Lage des Messbechers gegenüber dem Messwertgeber dadurch definiert ist, dass die Magnetkraft durch eine der Magnetkraft entgegengesetzte Gesamtgewichtskraft, die auf den Messbecher wirkt, ausgeglichen ist. Wie beschrieben kann anstelle einer Magnetkraft eine Federkraft, die durch ein zwischen Messbecher und Messwertgeber angeordnetes Federelement bereitgestellt wird, oder eine andere Lagerung, die eine zu der auf den Messbecher wirkenden Gewichtskraft entgegengesetzte Kraft als Rückstellkraft erzeugt, verwendet werden.

Der oder die Magnete können im Rahmen einer spritzgusstechnischen Herstellung des Messbechers mit ein-/ angegossen werden. Alternativ oder zusätzlich kann/können der oder die Magnete im Rahmen Herstellung des Messbechers formschlüssig eingepresst werden. Es ist auch möglich, dass der oder die Magnete im Rahmen der Herstellung des Messbechers eingeklebt werden.

Durch das Strömen des Urins durch den Messbecher hindurch kann die auf den Messbecher wirkende Gesamtgewichtskraft gegenüber der Magnetkraft erhöht sein, wodurch die Lage des Messbechers gegenüber dem Messwertgeber in Richtung der Gesamtgewichtskraft verschoben ist. Durch das Einströmen des Urins in eine Messkammer des Messbechers wird in diesem Fall das bestehende Gleichgewicht zwischen Magnetkraft und der auf den Messbecher wirkenden Gewichtskraft gestört und der Messbecher relativ zum Messwertgeber beispielsweise axial nach unten in Richtung der auf den Urin wirkenden Schwerkraft ausgelenkt, wobei die Auslenkung als Verschiebung detektiert wird.

Erfindungsgemäß ist der Messbecher innerhalb des Messwertgebers durch ein Führungsmittel derart geführt, dass eine Bewegung des Messbechers auf eine im Wesentlichen translatorische Bewegung beschränkt ist. Sofern der Becher eine Symmetrieachse aufweist, beispielsweise bei Rotationssymmetrie, kann durch das Führungselement lediglich eine Bewegung bzw. Verschiebung des Messbechers gegenüber dem Messwertgeber entlang der Symmetrieachse sichergestellt sein. Falls der Messwertgeber einen Behälter oder Rahmen für den Messbecher umfasst, der eine Symmetrieachse aufweist, kann der Messbecher durch das mindestens eine Führungselement im Messwertgeber derart geführt sein, dass bestimmungsgemäß lediglich eine Bewegung des Messbechers entlang der Symmetrieachse, also in axialer Richtung, möglich ist.

Das Führungsmittel umfasst in einer Ausführungsform eine oder mehrere Verbindungsstrebe(n), über die der Messbecher und der Messwertgeber miteinander verbunden sind. Das mindestens eine Führungsmittel kann über einen inneren und einen äußeren Teil verfügen, wobei beide Teile durch die eine oder mehreren Verbindungsstrebe(n) miteinander verbunden sind, wobei der äußere Teil mit dem Messwertgeber verbunden ist und der innere Teil mit dem Messbecher. Mit Vorteil wird durch die eine oder mehreren Verbindungsstrebe(n) eine Bewegung des inneren Teils zu dem äußeren Teil in axialer Richtung relativ zu dem äußeren Teil zugelassen, in radialer Richtung jedoch eine Bewegung des inneren Teils zu dem äußeren Teil verhindert.

Von dem Messwertgeber wird in einer weiteren Ausführungsform die Verschiebung des Messbechers gegenüber dem Messwertgeber und/oder eine Ableitung der Verschiebung nach der Zeit mittels eines Sensorelementes, bevorzugt berührungslos, detektiert. Von dem Sensorelement, das beispielsweise an dem Messwertgeber und/oder an dem Messbecher angeordnet ist, kann eine zu detektierende Lage/Position und/oder Bewegung des Messbechers gegenüber dem Messwertgeber detektiert werden.

Vorteilhaft wird eine axiale Lage/Position bzw. Bewegung des Messbechers in axialer Richtung im Messwertgeber erfasst wird. Die Erfassung der Lage/Position bzw. der Bewegung des Messbechers durch das Sensorelement kann berührungslos, beispielsweise optisch, erfolgen. Zusätzlich oder alternativ kann die Erfassung der Lage/Position bzw. der Bewegung des Messbechers mit Hilfe eines Magnetfeldsensors erfolgen. Als Sensorelement kann/können ein oder mehrere optische Maussensor(en) verwendet werden. Zur Erhöhung der Messgenauigkeit und Wartungsfreundlichkeit können mehrere Sensorelemente gleicher und/oder unterschiedlicher Bauart vorgesehen sein. Der gemeinsame und/oder einzelne Betrieb dieser Sensorelemente kann gemäß einem Ladezustand einer Stromversorgungseinheit, beispielsweise einer Batterie oder Akkuzelle, gesteuert sein. Für eine erhöhte Energieeffizienz können das oder die Sensorelement(e) zeitgesteuert sein. Beispielsweise kann ein Betrieb lediglich tagsüber oder zu Öffnungszeiten möglich sein

Die Verschiebung des Messbechers gegenüber dem Messwertgeber und/oder eine Ableitung der Verschiebung nach der Zeit sind/ist in einen Flusswert umrechenbar, wobei der Flusswert von dem Messwertgeber an eine Auswertungseinheit übertragbar ist. Die Übertragung anderer oder zusätzlicher Messdaten, beispielsweise die Zeit, der Ort der Messung oder ein Flusswertdiagramm über der Zeit, an die Auswertungseinheit ist möglich. Alternativ kann der Messwert lediglich die detektierte Verschiebung und/oder die Ableitung der Verschiebung nach der Zeit umfassen und die Umrechnung in einen Flusswert in der Auswertungseinheit erfolgen. Die Übertragung der Daten zwischen dem Messwertgeber und der Auswertungseinheit kann drahtlos und/oder drahtgebunden sowie ein- oder bidirektional erfolgen. Die Auswertungseinheit kann diesbezüglich zur Initialisierung der Messung einer Harnströmung, insbesondere über ein Touchpanel-Display, eingerichtet sein. Das Touchpanel Display erlaubt eine Interaktion mit der Vorrichtung, indem Bedienfunktionen ausgewählt und die Anzeigen dargestellt werden können. Alternativ oder zusätzlich kann ein Display nebst von dem Display getrennt angeordneten Bedientasten die Interaktion mit der erfindungsgemäßen Vorrichtung erlauben. Zur Wahrung der Privatsphäre des Patienten kann vor dem Display ein Blickschutzfilter positioniert sein.

Die Auswertungseinheit kann mit einem von der Auswertungseinheit separaten Messwertgeber Daten austauschen, wobei der Datenaustausch drahtlos und/oder kabelgebunden erfolgen kann. Es ist auch möglich, dass die Auswertungseinheit in den Messwertgeber integriert ist. In diesem Fall ist vorteilhafterweise ein Display und/oder eine Bedienungseinheit der Auswertungseinheit von dieser getrennt in Sicht- und/oder Bedienungsweite des Patienten angeordnet.

In die Auswertungseinheit kann ein Kartenlesegerät integriert und/oder angeschlossen sein. Alternativ oder zusätzlich kann in die Auswertungseinheit ein Kartenspeichergerät integriert und/oder angeschlossen sein. Die Integration oder der Anschluss eines Druckers in oder an die Auswertungseinheit ist ebenfalls möglich.

Die erfindungsgemäße Vorrichtung kann also neben einem Messbecher und einem Messwertgeber eine separate Auswertungseinheit umfassen. Vorteilhafterweise sind beide Komponenten durch entsprechende jeweils implementierte Technologien dazu in der Lage, drahtlos miteinander Daten auszutauschen. Denkbar ist eine Kommunikation über etablierte Nahbereichskommunikationstechnologien wie Bluetooth oder ZigBee, usw. wobei sowohl andere drahtlose Kommunikationstechnologien wie WLAN, Funk oder IR Kommunikation, als auch klassische kabelgebundene Technologien denkbar sind.

Die Auswertungseinheit ermöglicht es dem Anwender, mit dem System in Interaktion zu treten, indem Messungen initialisiert und Auswertungen angezeigt bzw. ausgegeben werden. Vorteilhafterweise weist die Auswertungseinheit ein Grafikdisplay mit integriertem berührungssensitivem Bildschirm (sog. Touch Panel) auf. Über ein geeignetes GUI (graphic user interface) ist es dem Anwender hierbei möglich, intuitiv Bedienfunktionen auszuführen, indem der Anwender situativ durch das Bedienkonzept angeleitet und unterstützt werden kann. Ferner lässt sich das GUI zielgruppen- und nutzerorientiert anpassen, so dass beispielsweise eine Bedienführung in verschiedenen Sprachen oder aber mit unterschiedlichen Schriftgrößen ermöglicht wird. Während die Verwendung verschiedener Sprachen die Bedürfnisse ausländischer Nutzergruppen adressiert, lassen sich über Schriftgrößen, Hell-/ Dunkelkontraste, Anordnung von Bedienelementen und Symboliken Vorteile für alte oder sehbehinderte Menschen erzielen. Ferner ist eine Sprach- und Videoausgabe denkbar, um dem Nutzer die Bedienung zu erläutern. Sofern gewünscht, können auf dem in der Auswertungseinheit integrierten Bildschirm Werbebotschaften während der Messpausen dargestellt werden, so dass hierüber ein Refinanzierungsmodell für die Anschaffung und den Betrieb des Messsystems dargestellt werden kann.

Vorteilhafterweise ist die Auswertungseinheit in ein robustes Edelstahlgehäuse oder aber hinter eine Edelstahlblende eingebaut, wobei zur Vermeidung unerwünschter Effekte der Abschattung des zur drahtlosen Kommunikation mit dem Messwertgeber verwendeten Feldes auch eine vollständige oder wenigstens partielle Ausführung in entsprechend robusten Kunststoffen oder vergleichbaren Materialien denkbar ist. Die Verwendung hochwertiger Materialien hat sowohl Vorteile hinsichtlich Langlebigkeit und Vandalismusschutz und vermittelt zugleich einen edlen, werthaltigen und gleichzeitig zuverlässigen Eindruck. Aufgrund ihrer Kompaktheit ist die Auswertungseinheit in einer ergonomisch günstigen Position, beispielsweise auf Brusthöhe im Fall eines Urinals, dem Nutzer zugewandt, an einer Wand zu befestigen. Denkbar sind hier sowohl Aufputz- wie Unterputzmontagen, wobei die Aufputzmontage in einem Gehäuse primär zur Nachrüstung in bestehende Sanitäranlagen intendiert ist, die Unterputzmontage mit einer Geräteblende hingegen insbesondere für eine Neuausrüstung einer Klinik oder urologischen Praxis vorteilhaft erscheint. Zur Energieversorgung sind sowohl Geräteversionen denkbar, die eine externe Stromversorgung benötigen, ebenfalls sind Versionen denkbar, die über eine autonome Energieversorgung (insbesondere Batterien oder Akkus) verfügen. Obgleich eine Auswertungseinheit ohne eine externe Energieversorgung zunächst nicht vorteilhaft erscheint, so ist dies aus Gründen der problem- und aufwandlosen Nachrüstbarkeit in bestehende Sanitäranlagen, insbesondere im nichtmedizinischen Umfeld, durchaus eine interessante Option. Um die Notwendigkeit für Batterie- bzw. Akkuwechsel in diesem Fall gering zu halten, ist ein sog. Sleepmodus sinnvoll, bei dem das System bei Nichtverwendung in einen Stromsparmodus versetzt wird und beispielsweise durch einen Näherungssensor wieder in einem aktiven Betriebsmodus versetzt wird.

Zur Wahrung der Privatsphäre für den nichtmedizinischen Einsatz im Rahmen eines Screenings an öffentlichen Orten ist es insbesondere vorteilhaft, wenn konstruktive oder sonstige technologische Maßnahmen vorgesehen werden, um den Blickwinkel auf das Display stark zu begrenzen. Dies soll bewirken, dass weitere Anwesende Personen nicht oder zumindest sehr stark eingeschränkt den Bildschirm betrachten können. Wie bereits beschrieben kann in einer vorteilhaften Ausgestaltung der Erfindung dies durch Aufsetzen bzw. durch Integration eines entsprechenden Blickschutzfilters erfolgen, welcher beispielsweise durch das Unternehmen 3M entwickelt wurde (Vikuti™ Blickschutzfilter). Die genannten Filter bestehen aus Kunststoff und basieren auf der Mikrolamellen- Technologie. Hierbei werden feinste schwarze, nicht reflektierende Lamellen, über ein Dutzend pro Millimeter, hochgenau positioniert. Wenn ein bestimmter seitlicher Blickwinkel überschritten wird, führen die Lamellen zu einer vollständigen Abschattung des Bildes.

Insbesondere zum Einsatz in einem klinischen bzw. medizinischen Umfeld ist die Integration eines Kartenlesegerätes in die Auswertungseinheit vorteilhaft. In diesem Szenario wird der Patient bei der Aufnahme in der medizinischen Einrichtung mit einer personalisierten Chipkarte ausgestattet. Zum Starten einer Untersuchung führt der Patient die Chipkarte in das Kartenlesegerät ein. Sinnvollerweise wird über einen Dialog auf dem Display der Patient aufgefordert, sich als die auf der Karte hinterlegte Person zu identifizieren. Sofern die medizinische Einrichtung über eine entsprechende Hard- und Softwarearchitektur verfügt, ist es denkbar, dass die folgende Messung aufgrund Unterstützung geeigneter Kommunikationsschnittstellen umgehend in der Patientenakte elektronisch gespeichert wird. Hierzu weist die Auswertungseinheit vorteilhafterweise einen LAN und/ oder einen WLAN Anschluss auf. Da bei diesem denkbaren Szenario lediglich ein Lesen, nicht jedoch ein Schreiben auf die Chipkarte notwendig ist, kann zum Zwecke der Kostenersparnis der Nutzer alternativ seine Versichertenkarte in das Lesegerät einführen und hiermit über die Zuordnung seines auf der Karte gespeicherten Namen zu einem vorhandenen Datensatz das Abspeichern der nachfolgend im Rahmen der Messung gewonnenen Daten initiieren.

Sofern keine elektronische Patientenverwaltung und Datenarchivierung in der medizinischen Einrichtung verfügbar sind, kann die Messung alternativ auf der erwähnten personalisierten Karte abgespeichert werden. An einem separaten Terminal kann bei diesem Setup über ein gesondertes Kartenlesegerät, beispielsweise stationiert am Empfangstresen der urologischen Praxis, der Karteninhalt wieder ausgelesen und beispielsweise ausgedruckt oder auf einem Server o.ä. abgespeichert werden.

Im nichtmedizinischen Umfeld, insbesondere zum Zwecke eines Screenings ist eine personalisierte Messdatenerfassung weder medizinisch notwendig noch aus Gründen des Datenschutzes angezeigt. Hier ist es vielmehr sinnvoll, dem Nutzer im Fall einer diagnostizierten potenziellen Erkrankung oder noch allgemeiner gesprochen einer Auffälligkeit einen Ausdruck des Flowprofils, sowie die hieraus ermittelten relevanten Messparameter wie Spitzenfluss, mittlerer Fluss, Miktionszeit usw. mitzugeben, beispielsweise mit Hilfe eines in der Auswertungseinheit integrierten oder an diese angeschlossenen Thermodruckers. Ferner kann es angezeigt sein, dem Anwender über einen entsprechenden aufgedruckten Hinweistext den Besuch einer urologischen Praxis anzuraten und den Ausdruck dorthin mitzunehmen. Der Ausdruck hat einerseits für den Anwender den Erinnerungscharakter, einen Facharztbesuch einzuplanen, andererseits kann der nun aufgesuchte Facharzt anhand der ihm präsentierten Daten eine erste Bewertung der Messung vornehmen und die nächsten diagnostischen Schritte einleiten. Als positiven Seiteneffekt kann der Ausdruck zudem dazu verwendet werden, Werbung abzudrucken und hierüber die Anschaffung und den Betrieb eines solchen Harnflussmesssystems insbesondere im nichtmedizinischen Umfeld zu refinanzieren.

Der Messwertgeber, welcher mit der Auswertungseinheit in einer vorteilhaften Version der Erfindung drahtlos kommuniziert, umfasst beispielsweise als Sensorelement den "eigentlichen" Sensor, welcher die Wandlung des variablen Harnflusses in eine elektrisch auswertbare und auf diese Weise gegebenenfalls elektronisch und/oder mittels Software verarbeitbare Messgröße vornimmt, eine Messelektronik mit integriertem Modul zur drahtlosen Kommunikation, sowie eine Stromversorgung. Alternativ oder zusätzlich zu einer internen Stromversorgung des Messwertgebers kann eine externe Stromversorgung für den Messwertgeber vorgesehen werden.

Der Messbecher umfasst in einer weiteren Ausführungsform einen Strömungswiderstand, der in dem Messbecher derart angeordnet ist, dass der Urin der Gewichtskraft folgend durch den Strömungswiderstand hindurch strömen kann und beim Strömen des Urins durch den Strömungswiderstand hindurch der Urin gestaut werden kann zur Sammlung des Urins in dem Messbecher. Der Messbecher kann eine Messkammer, in die der Urin geleitet wird, den Strömungswiderstand, durch den der Urin der auf den Urin wirkenden Gewichtskraft folgend hindurchfließt, und einem Ausflussteil, durch den der Urin in ein Urinal bzw. in ein WC Becken fließt, umfassen. Der Messbecher ist also so ausgeführt, dass er von dem Urin durchströmt werden kann und beim Durchströmen zumindest ein Teil des Urins in dem Messbecher gesammelt werden kann. In einer einfachen Ausführung kann der Messbecher als Behältnis mit zwei sich gegenüberliegenden Öffnungen unterschiedlichen Durchmessers, beispielsweise als an zwei sich gegenüberliegenden Seiten offener Kegelstumpf, ausgeführt sein. Die Öffnung mit dem größeren Durchmesser dient dem Zufluss des Urins und die Öffnung mit dem kleineren Durchmesser dient dem Abfluss und der Sammlung des Urins. Alternativ kann der Messbecher als offener Zylinder mit einem den Urin beim Durchströmen des Messbechers stauenden Netz oder Filter innerhalb des Zylinders ausgeführt sein.

Besonders vorteilhaft ist es, wenn abhängig von der Flüssigkeitsströmung und dem Strömungswiderstand sich der Urin in der Messkammer des Messbechers oder in dem Messbecher selbst beim Durchströmen des Messbechers staut. Beispielsweise kann der Strömungswiderstand eine Querschnittsfläche aufweisen, durch die hindurch der Urin aus dem Messbecher herausströmen kann, die proportional ist zu einem Füllstand des Urins in dem Messbecher. Ein solcher Strömungswiderstand kann kegelstumpfförmig ausgestaltet sein und eine sternenförmige Querschnittsfläche derart aufweisen, dass bei einem niedrigen Füllstand des Urins im Messbecher weniger Urin abfließen kann als bei einem Füllstand des Urins im Messbecher, der gegenüber dem niedrigen Füllstand erhöht ist. Dem Urin wird bei geringem Füllstand in einer Messkammer des Messbechers eine kleine Querschnittsfläche zur Verfügung gestellt, um in den Ausflussteil des Messbechers zu gelangen. Bei hohem Füllstand in der Messkammer wird dem Urin eine große Querschnittsfläche zur Verfügung steht, um in den Ausflussteil des Messbechers zu gelangen. Auf diese Weise kann bei gegebener Verschiebung eine erforderliche Messgenauigkeit über einen größeren Flusswertbereich sichergestellt werden.

Ein Auffangbehälter zum Sammeln des Urins kann in einer weiteren Ausführungsform vorgesehen sein, über den der gesammelte Urin in den Messwertgeber geleitet werden kann. Der Auffangbehälter kann aus einem flexiblen Material bestehen, beispielsweise zur Anpassung eine gegebene Kontur eines Urinals oder WC Beckens. Der Auffangbehälter ist mit Vorteil so geformt, dass dieser in ein handelsübliches Urinal oder WC Becken eingesetzt werden kann.

Der Messwertgeber zum Messen der Harnströmung ist vorteilhafterweise unterhalb des Auffangbehälters in Richtung der Schwerkraft des Urins positioniert, wenn der Messbecher von Urin durchströmt wird. Der Messbecher kann in den Messwertgeber einsetzbar ausgeführt sein. Auf diese Weise kann ein Ausbau des Messwertgebers vermieden werden, wenn lediglich der Messbecher, beispielsweise zur Reinigung und/oder Wartung, entnommen werden soll.

Zwischen dem Auffangbehälter und dem Messwertgeber kann in einer weiteren Ausführung der Erfindung ein den Messwertgeber abdeckender Deckel angeordnet sein, in den von dem Auffangbehälter gesammelter Urin geleitet werden kann. Der Auffangbehälter ist vorteilhafterweise so zum Deckel angeordnet, dass während der Messung der vom Auffangbehälter gesammelte Urin am tiefsten Punkt des Auffangbehälters in Richtung der auf den Urin wirkenden Gewichtskraft in den Deckel geleitet wird.

Der Deckel kann mindestens einen Schlitz oder Lock, bevorzugt jedoch eine Mehrzahl von Schlitzen und/oder Löcher, aufweisen, die ein Eindringen von Fremdstoffen in den Messwertgeber verhindern sollen. Der Urin wird vorteilhafterweise durch Führungskanäle im Deckel zum Mittelpunkt desselben geleitet bzw. geführt.

Der Deckel ist in einer weiteren Ausführung derart geformt, dass beim Leiten des von dem Auffangbehälter gesammelten Urins in den Deckel der in Richtung der Gewichtskraft des Urins durch eine Öffnung oder mehrere Öffnungen im Deckel vertikal abfließende Urin über ein Prallelement, das bevorzugt als Teil des Deckels ausgebildet ist, in eine im Wesentlichen horizontale Strömungsrichtung umgeleitet ist. Der senkrecht durch den mindestens einen Schlitz oder Loch, bevorzugt jedoch mehrere Schlitze und/oder Löcher abfließende Urin wird also über ein Prallelement in eine nahezu horizontale Strömungsrichtung umgeleitet.

Das Prallelement kann zu diesem Zweck rotationssymmetrisch ausgebildet sein und einen sich vergrößernden Durchmesser in einer Richtung aufweist, die beim Abfließen des Urins der Richtung der Gewichtskraft des abfließenden Urins entspricht. Das Prallelement ist also rotationssymmetrisch aufgebaut und weist einen sich nach unten verbreiternden Oberflächenverlauf während der Messung auf. Das Prallelement kann hierbei die Form eines Kegels oder Trichters aufweisen, der sich in eine Richtung entgegen der Richtung der auf den Urin wirkenden Schwerkraft verjüngt. Wie beschrieben ist das Prallelement vorteilhaft als Bestandteil des Deckels ausgeführt.

Der Deckel ist vorteilhafterweise so ausgeführt, dass durch diesen Deckel nach Einsetzen in den Messwertgeber der äußeren Teil des mindestens einen beschriebenen Führungsmittels geklemmt ist und damit eine ungewollte Ortsveränderung des äußeren Teils dieses Führungsmittels verhindert. Zu diesem Zweck kann/können in dem Deckel eine Klemmstrebe oder Klemmstreben vorgesehen sein. Die Klemmstrebe(n) verhindert(n) nicht nach Einsetzen des Deckels in den Messwertgeber nicht nur eine Verrutschen des Messbechers gegenüber dem Messwertgeber, sondern fixieren vorteilhaft den Deckel am Messwertgeber.

Erfindungsgemäß ist zudem ein Verfahren zur Messung einer Harnströmung eines Patienten vorgesehen, bei dem ein Messbecher gegenüber einem Messwertgeber derart angeordnet wird, dass eine Verschiebung des Messbechers gegenüber dem Messwertgeber als Maß für ein Volumen von in dem Messbecher gesammeltem Urin detektiert werden kann, wobei die Verschiebung von einer Erhöhung eines Gesamtgewichtes des Messbechers bewirkt wird, die beim Strömen des Urins durch den Messbecher hindurch dem Gewicht des im Messbecher gesammelten Urins entspricht, der Urin durch den Messbecher hindurch strömt, und die Verschiebung des Messbechers gegenüber dem Messwertgeber beim Strömen des Urins durch den Messbecher hindurch als Maß für das Volumen des im Messbecher gesammelten Urins detektiert wird.

Weitere Ausführungsbeispiele sowie Vorteile der Erfindung werden nachfolgend anhand der Figuren erläutert. Zur besseren Anschaulichkeit wird in den Figuren auf eine maßstabs-/ oder proportionsgetreue Darstellung verzichtet. In den Figuren bezeichnen, sofern nicht anders angegeben, gleiche Bezugszeichen gleiche Teile mit gleicher Bedeutung. Es zeigen:
Fig. 1 schematisch im Querschnitt einen Auffangbehälter 1, der in einen erfindungsgemäßen Messwertgeber 4 mit einem Messbecher 11 zur Wägemessung einer Harnströmung mündet,
Fig. 2 in einer Draufsicht schematisch eine Messkammer 13 des Messbechers 11, wobei in dem Messbecher 11 ein Strömungswiderstand 14 angeordnet ist, der eine nichtlineare Charakteristik aufweist,
Fig. 3a in einer schematischen Seitenansicht den in Fig. 2 dargestellten Messbecher 11 und eine auf den Strömungswiderstand 14 projizierte Fläche 19a bei einem mittleren Füllstand 18a der Messkammer 13,
Fig. 3b in einer weiteren Seitenansicht den in Fig. 2 dargestellten Messbecher 11 und eine auf den Strömungswiderstand 14 projizierte Fläche 19b bei einem im Vergleich zu dem in Fig. 3a dargestellten Füllstand 18a hohen Füllstand 18b der Messkammer 13,
Fig. 3c in einer weiteren Seitenansicht den in Fig. 2 dargestellten Messbecher 11 und eine auf den Strömungswiderstand 14 projizierte Fläche 19c bei einem im Vergleich zu dem in Fig. 3a dargestellten Füllstand 18a niedrigen Füllstand 18c der Messkammer 13,
Fig. 4 schematisch den Aufbau der oberen Führung 21 des Messbechers 11 in einer Draufsicht, wobei der dargestellte Aufbau ebenso zur Darstellung der unteren Führung 20 des Messbechers 11 dienlich ist.

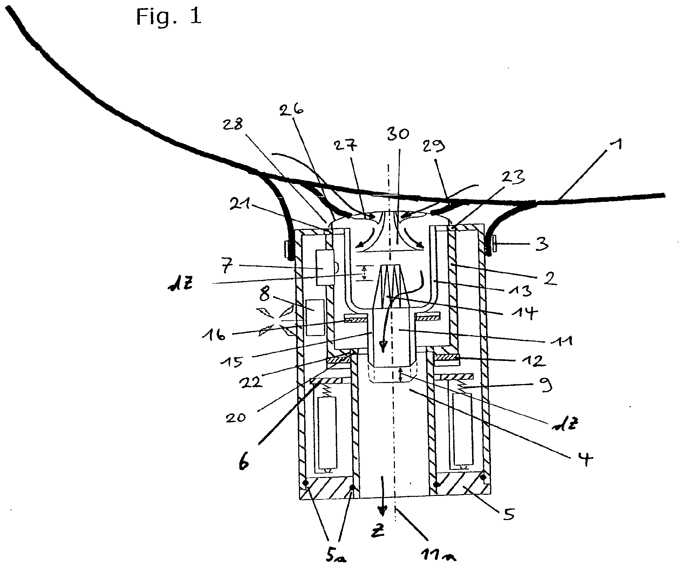

Der Messwertgeber 4, welcher ein Gegenstand dieser Erfindung ist, wird nachfolgend anhand von Fig. 1 näher beschrieben:
Ein Auffangbehälter 1, beispielsweise bestehend aus einem flexiblen gummiartigen Material, ist derart dimensioniert, dass er sich verformbar entlang der Innenwände eines Urinal einsetzen lässt. Abhängig von den am Markt befindlichen Urinalen sind sowohl ein universeller Auffangbehälter 1, als auch eine Mehrzahl von urinalspezifischen Auffangbehältern denkbar. Ähnlich einem Trichter sich nach unten verjüngend, mündet der Auffangbehälter 1 in einen senkrechten rotationssymmetrischen Behälter 2, wobei der rotationssymmetrische Behälter 2 mit dem Auffangbehälter 1 wasserdicht miteinander verbunden ist. Die Verbindung kann auf unterschiedliche Weise technisch realisiert werden, beispielsweise durch Aufziehen des flexiblen Auffangbehälters 1 auf den rigiden rotationssymmetrischen Behälter 2, wobei eine Sicherung der Verbindung durch Zuhilfenahme einer Schlauchschelle 3 erfolgt. Bevorzugt besteht die Schlauchschelle 3 aus einem korrosionsunempfindlichen Material wie beispielsweise Edelstahl.

Der rotationssymmetrische Behälter 2, der auch eine andere Form aufweisen kann, bildet die innere Gehäusewandung des bevorzugt rotationssymmetrisch aufgebauten Messwertgebers 4. Der Messwertgeber 4 ist einer denkbaren bevorzugten Ausführungsform mit einem Gehäusedeckel 5 wiederverschließbar verschlossen und mit einer umlaufenden Dichtung 5a gegen das Eindringen von Flüssigkeiten hermetisch abgedichtet. Der Gehäusedeckel 5 und die umlaufende Außenwandung des Messwertgebers 4 weisen in einer besonders vorteilhaften Ausführungsform ein Gewinde auf, mit dem beide Teile miteinander verschraubt werden können (nicht dargestellt, gleichwohl kann die Befestigung beider Teile mit- und zueinander aber beispielsweise auch unter Zuhilfenahme von gesonderten bevorzugt rostfreien Schrauben o.Ä. realisiert werden. Ebenso ist in einer alternativen Bauform auch ein Vergießen des Messwertgebers mit Gießharz, Silikon usw. denkbar, so dass sich die Notwendigkeit des Verschließens mit einem Deckel erübrigt. Im Inneren des Messwertgebers befindet sich eine bevorzugt rund ausgestaltete Platine 6, welche im Inneren eine Aussparung für den rotationssymmetrischen Behälter 2 aufweist. Andere Platinengeometrien sind möglich. Auf dieser Platine 6 sind ein Sensorelement 7, eine geeignete Auswertungs- und Signalaufbereitungselektronik, sowie Bauelemente zur Datenverarbeitung (nicht dargestellt) enthalten, zudem elektronische Bauelemente zur drahtlosen Kommunikation 8 mit der Auswertungseinheit. Ferner sind Kontakte 9 zur Stromabnahme aus der bzw. den im Messwertgeber 4 eingesetzten (und sofern es sich um eine Mehrzahl selbiger handelt) rotationssymmetrisch um den Behälter 2 angeordneten Batterie(n) 10 vorhanden. Das Sensorelement 7 dient der Positionserfassung eines Messbechers 11, welcher in den Messwertgeber 4 eingesetzt werden kann, bzw. der Detektion einer Verschiebung dZ des Messbechers 11 gegenüber dem Messwertgeber 4. In einer bevorzugten Ausführungsform der Erfindung ist das Sensorelement 7 ein optischer Bewegungs- bzw. Positionssensor, wobei insbesondere ein Sensortyp, wie er in Computereingabegeräten (sog. "optischer Maussensor") zum Einsatz kommt, verwendet werden kann. Beispielsweise kann der Sensor PAN301-204 der Firma PixArt Imaging Inc. als Sensorelement 7 verwendet werden. Alternativ sind andere berührungslose Sensoren als Sensorelement 7 verwendbar, beispielsweise Magnetfeldsensoren.

Im Inneren des rotationssymmetrischen Behälters 2 ist ein Magnet 12, bevorzugt ein Ringmagnet angeordnet. In einer alternativen Bauform ist eine Mehrzahl von Magneten denkbar, welche rotationssymmetrisch umlaufend zu dem Behälter 2 mit identischer Polungsrichtung, beispielsweise alle Südpole nach unten, angeordnet werden.

Der Messbecher 11 ist beispielsweise als rotationssymmetrisches Kunststoffformteil mit einer Symmetrieachse 11a in Z-Richtung ausgeführt und weist eine Messkammer 13, einen Strömungswiderstand 14 und einen Ausflussteil 15 auf. Ferner ist in dem Messbecher eine Mehrzahl von Magneten 16 angeordnet, bevorzugt eingepresst oder auch ein-/angespritzt bzw. geklebt. Alternativ ist auch ein einzelner Ringmagnet denkbar, wobei aus Kostengesichtspunkten diese Ausführungsform Nachteile gegenüber der Umsetzung mit Einzelmagneten aufweist, bevorzugt in Form von drei Einzelmagneten um je 120° in einer Ebene senkrecht zur Symmetrieachse 11a in Z-Richtung versetzt, wobei die Ebene durch eine x-Richtung und eine Y-Richtung aufgespannt wird. Unabhängig von der Formgebung und der Anzahl der Magnete ist die Polarität der bzw. des Magnete(n) 16 entgegengesetzt zu dem im Behälter 2 angeordneten Magneten 12. In der Folge stoßen sich Messbecher und Behälter 2 voneinander ab. Der Gewichtskraft folgend, ist der in den senkrecht angeordneten Messwertgeber 4 eingesetzte Messbecher 11 bestrebt, nach unten zu sinken. Gleichzeitig sind beide Komponenten aufgrund der Magnetkräfte bestrebt, sich voneinander zu entfernen. Abhängig vom Gewicht des Messbechers 11 ergibt sich eine definierte Ruhelage bzw. Ruheposition, in der sich beide Kräfte ausgleichen. Der Messbecher 11 schwebt bei Erreichen dieser Ruhelage in dem Messwertgeber 4. Um eine hohe Sensitivität, insbesondere für kleine Flows zu erzielen, ist es vorteilhaft, das Gewicht des Messbechers sehr gering zu halten, beispielsweise kleiner 15g. Hierdurch sind zudem weniger starke Magnete 16, 12 notwendig, was sich günstig auf die Herstellkosten, insbesondere des Messbechers 11, auswirkt.

Der bei einer Miktion abgesetzte Urin wird durch geeignete Maßnahmen, welche weiter unten erläutert werden, definiert in die Messkammer 13 des Messbechers 11 geleitet. Durch den im Messbecher integrierten Strömungswiderstand 14 staut sich nun der Urin in der Messkammer 13 und vergrößert das Gesamtgewicht des Messbechers 11, so dass dieser aus seiner Ruhelage heraus nach unten sinkt und um eine Verschiebung dZ verschoben wird. Die Wegänderung in Form der Verschiebung dZ des Bechers 11 gegenüber dem Messwertgeber 4 lässt sich nun mit dem Sensorelement 7 detektieren, in einen Volumenstrom durch die Elektronik nebst Software umrechnen und die Information mit der Sendeeinheit 8 an die Auswertungseinheit senden, wo sie weiter ausgewertet und zur Anzeige gebracht wird.

Der Strömungswiderstand 14 kann hierbei auf verschiedenste Art realisiert sein. Die einfachste Lösung ist schlicht eine definierte Blende, durch die der Harn aus der Messkammer 13 in den Ausflussteil 15 fließt und nach dessen Passieren den Messwertgeber 4 verlässt und somit in das Urinal abfließt. Je kleiner der Blendendurchmesser, desto höher die Sensitivität für kleine Flows. Dieses an sich erstrebenswerte Ziel erweist sich jedoch insofern in der Umsetzung als nachteilig, als dass hierdurch der Messbereich für den maximal messbaren Fluss stark limitiert wird. Wenn dieser maximale Messbereich überschritten wird, kann sowohl der Messbecher 11 überlaufen, oder aber der Becher 11 kann durch die Magnetkräfte nicht mehr in der Schwebe gehalten werden und berührt in der Folge den Behälter 2. Beides sind Effekte, die die Messgenauigkeit empfindlich stören würden. Das mechanische Auftreffen des Messbechers 11 auf den Behälter 2 könnte hypothetisch durch Einsatz stärkerer Magnete 12, 16 im Prinzip abgestellt werden, hätte nun jedoch zur Folge, dass die Sensitivität für kleine Flows drastisch abnehmen würde, da eine geringe Flüssigkeitsmenge nur noch eine geringe Verschiebung dZ bzw. Auslenkung des Messbechers 11 zur Folge hätte. Folglich ist dies keine befriedigende Maßnahme. Ferner verbleibt die Problematik, dass der Messbecher bei hohen Flows weiterhin überlaufen kann. Während der bevorzugte Aufbau des Messwertgebers 4 hiervon grundsätzlich keinen Schaden davon trägt, da der überlaufende Urin zwischen Messbecher 11 und dem rotationssymmetrischen Behälter 2 der Schwerkraft folgend aus dem Messwertgeber 4 in das Urinal abfließen kann, verbleibt die Problematik, dass die korrekte Messung hoher Flussraten typischerweise eine weite Öffnung der Blende verlangt.

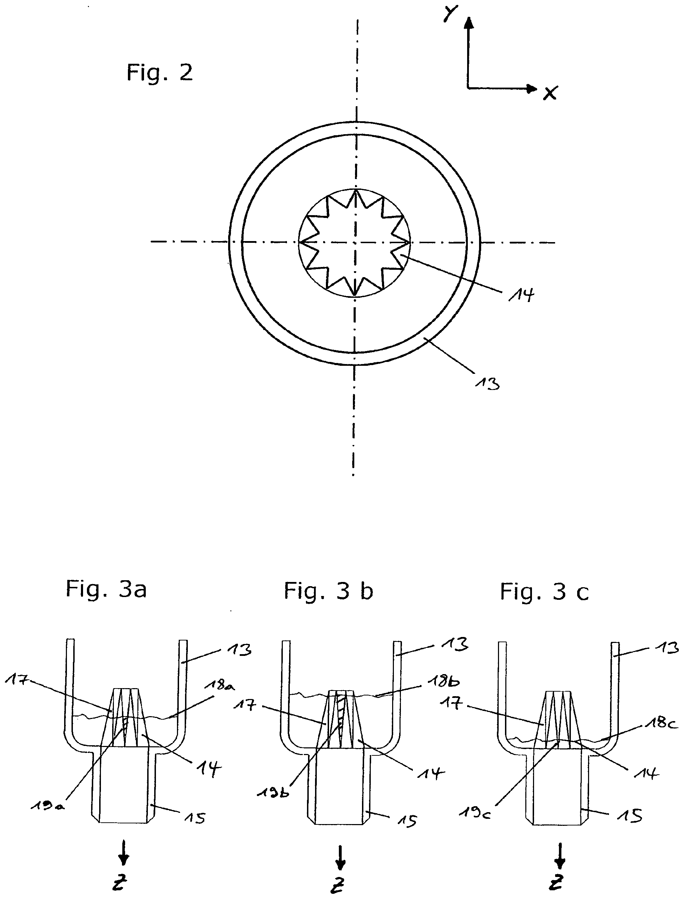

Der beschriebene Zielkonflikt aus Forderung nach einer engen Blende zur Messung kleiner Flows und weiter Blende zur Messung hoher Flows wird erfindungsgemäß durch eine Blende mit nichtlinearer Charakteristik, d.h. einer Charakteristik, bei der der Strömungswiderstand mit höheren Flows kleiner wird, gelöst. Die Fig. 2 bis 3.c zeigen eine beispielhafte Blendengestaltung, wobei sich die Erfindung ausdrücklich nicht auf ausschließlich diese hier ausgeführte mögliche Ausführungsform des zugrunde liegenden erfinderischen Gedankens beschränkt.

Fig. 2 zeigt eine Draufsicht auf die Messkammer 13 und auf einen Strömungswiderstand 14 als Beispiel für einen Strömungswiderstand, der eine nichtlineare Charakteristik aufweist. Der Strömungswiderstand weist in der Draufsicht ein sternförmiges Profil auf.

Fig. 3a zeigt denselben Strömungswiderstand 14 in einer Seitenansicht. Es ist erkennbar, dass die Grundform einem Kegelstumpf entspricht, wobei der Stumpf durch zackenförmige Ausnehmungen 17 bis zum Grund der Messkammer 13 unterbrochen ist. Die Figur zeigt zudem einen mittleren Füllstand 18a der Messkammer 13 und die projizierte Fläche 19a einer beispielhaft gewählten zackenförmigen Ausnehmung des kegelstumpfförmigen Strömungswiderstandes 14, durch die der Harnstrom beim Füllstand 18a in den Ausflussteil 15 gelangen kann.

Fig. 3b zeigt eine Seitenansicht des Strömungswiderstandes 14, der einen im Vergleich zu dem in Fig. 3a dargestellten Füllstand 18a hohen Füllstand 18b aufweist. Es ist erkennbar, dass die projizierte Fläche 19b der identischen zackenförmigen Ausnehmung wie in Fig. 3a größer ist als die projizierte Fläche 19a, die in Fig. 3a dargestellt ist.

Fig. 3c zeigt schließlich eine Seitenansicht des Strömungswiderstandes 14, jedoch mit einem im Vergleich zu dem in Fig. 3a dargestellten Füllstand 18a niedrigen Füllstand 18c. Die projizierte Fläche 19c ist klein im Vergleich zu der in Fig. 3a dargestellten projizierten Fläche 19a.

Je höher der Volumenstrom ist, desto höher wird sich die Flüssigkeit in der Messkammer 13 stauen. Je höher sich die Flüssigkeit staut, desto größer wird die Blendenfläche, der Strömungswiderstand nimmt somit ab.

Die bereits erwähnte magnetische Lagerung erweist sich als besonders vorteilhaft, da mechanische Reibungseffekte zwischen der Messkammer 13 und dem Behälter 2 aufgrund der Lagerung selbst, die beispielsweise bei einer Kugellagerung auftreten würden, vermieden werden, welche sich andernfalls als nichtlineare Messfehler, insbesondere bei kleinen Flows, bemerken machen würden. Die magnetische Lagerung führt hingegen zu einem Luftspalt zwischen der Messkammer 13 und dem Behälter 2 wie in Fig. 1 dargestellt, so dass eine Berührung zwischen Messkammer 13 und Behälter 2 ausgeschlossen ist und lediglich eine vernachlässigbar kleine Luftreibung zwischen der Messkammer 13 und dem Behälter 2 auftritt. Allerdings gilt die Annahme einer weitestgehend reibungsfreien Lagerung nur dann, wenn eine vollständig lotrechte Ausrichtung, also ein Ausrichtung der Symmetrieachse 11a in vertikaler Richtung, sichergestellt werden kann und sich Messbecher 11 und der Behälter 2 nicht berühren können. Dies kann in der Praxis nicht immer sichergestellt werden, zumal der in die Messkammer 13 einfließende Urin auch seitliche (Impuls-) Kräfte auf den Messbecher 11 übertragen und diesen somit aus seiner lotrechten Lage bringen kann, so dass die Reibung zwischen Messbecher 11 und rotationssymmetrischem Behälter 2 zunehmen und zu Nichtlinearitäten im Strömungsverlauf führen kann.

Die genannten Nichtlinearitäten sind insbesondere durch wechselnde Haft-/ Gleitreibungseffekte begründet, welche einen treppenförmigen Verlauf des Messsignals hervorrufen können.

Um genannte verbleibende Reibungseffekte möglichst zu verhindern, ist der Messbecher 11 sowohl am unteren Ende mit einem Kunststoffformteil, nachfolgend als untere Führung 20 bezeichnet, als auch mit einem weiteren Kunststoffformteil, nachfolgend obere Führung 21, ausgestattet.

Beim Einsetzen des Messbechers 11 in den rotationssymmetrischen Behälter 2 stützt sich die untere Führung 20 seitlich gegen die Innenwand des Messwertgebers 4 ab und liegt an einem Bund 22 am unteren Ende des rotationssymmetrischen Behälters 2 wie in Fig. 1 dargestellt an.

Die obere Führung 21 sitzt formschlüssig in einem weiteren Bund 23 am oberen Ende des rotationssymmetrischen Behälters 2 wie ebenfalls in Fig. 1 dargestellt. Formschlüssig bedeutet, dass der Behälter 2 eine Aussparung / Fräsung an der Stirnseite aufweist, in welche die Führung 21 eingelegt werden kann.

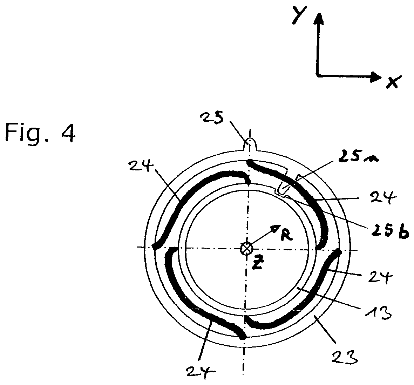

Fig. 4 zeigt den prinzipiellen Aufbau der oberen Führung 21 in einer Draufsicht, wobei das Prinzipbild ebenso zur Beschreibung der unteren Führung 20 dienlich ist: Im Inneren ist die rotationssymmetrische Messkammer 13 des Messbechers 11 erkennbar, außen ein Klemmring 23 mit einer hervorstehenden, radial nach außen ausgerichteten Nase 25. Der Klemmring 23 weist eine hervorstehende, radial nach innen zum Messbecher 11 ausgerichtete weitere Nase 25a auf, die in einer Ausnehmung 25b in Form einer länglichen Vertiefung oder Nut in Z-Richtung an einer Außenseite des Messbechers 11 geführt ist.

Die Messkammer 13 und der Klemmring 23 werden durch eine Mehrzahl von Verbindungsstreben 24 miteinander verbunden. Diese Streben 24 dienen dazu, die Messkammer 13 relativ zum Klemmring 23 axial zu führen und zu verhindern, dass die Messkammer den rotationssymmetrischen Behälters 2 berühren kann.

Da die Verbindungsstreben 24 nicht auf kürzestem Wege Messkammer 13 und Klemmring 23 miteinander verbinden, sondern die Verbindungspunkte der Streben 24 zu dem Klemmring 23 gegenüber den Verbindungspunkten dieser Streben 24 zu der Messkammer 13 in einer zur axialen Richtung Z und zur radialen Richtung R senkrechten Richtung zueinander versetzt (entlang einer Innenwandung des Klemmrings 23) angeordnet sind, ergibt sich bei axialer Verschiebung dZ bzw. Auslenkung des Messbechers nach unten im Messwertgeber 4 eine reversible Verbiegung der Verbindungsstreben 24. Die Verbindungsstreben 24 agieren in dieser Ausgestaltung als flexible Blattfedern mit geringer Federsteifigkeit. Abhängig von der Länge, der Anzahl, der Form und der Materialstärke der Verbindungsstreben 24 lässt sich die Federsteifigkeit beeinflussen, bevorzugt minimieren, sowie die maximale Auslenkbarkeit der Messkammer relativ zum Klemmring einstellen. Bei geeigneter, bevorzugt durch geeignete FEM (Finite Elemente Methode) Softwareprogramme simulierte und gleichzeitig optimierte, Parameterwahl in der Ausgestaltung der Verbindungsstreben 24, lässt sich sicherstellen, dass die Auslenkung in Form der Verschiebung dZ der Messkammer 13 relativ zum Klemmring 23 nur in jenen Grenzen erfolgen kann, in denen die Federkennlinie linear ist und es somit zu keiner bleibender Materialverformung der Verbindungsstreben 24 in Folge von Überdehnung derselben kommt. Die nach außen ausgerichtete Nase 25 dient hierbei der Aufnahme einer möglichen Tangentialkraft, die bei einer Verschiebung dZ bzw. Auslenkung von der Messkammer 13 auf den Klemmring 23
übertragen werden kann. Die in der Ausnehmung 25b im Messbecher 11 geführte nach innen ausgerichtete weitere Nase 25a dient der Aufnahme etwaiger Tangentialkräfte, die ohne die weitere Nase 25a zu einer Rotation des Messbechers 11 führen könnten. Sofern die Eigenschaften der Lagerung der Messkammer 13 gegenüber dem Behälter 2 unabhängig von einer Rotation der Messkammer 13 gegenüber dem Behälter 2 sind, beispielsweise durch Formung der Magnete 12 und 16 jeweils als Ringe mit über dem Umfang konstanten Querschnittsmaßen, können/kann die weitere Nase 25a und/oder die Nase 25 entfallen und eine Rotation des Messbechers 11 gegenüber dem Behälter 2 zugelassen werden. Auf diese Weise können/kann die obere Führung 21 und/oder die untere Führung 20 einfach realisiert werden und eine Reibungskomponente zwischen der Nase 25a und der Messkammer 13 vermieden werden.

Der prinzipielle Aufbau der unteren Führung 20 ist wie erwähnt analog, so dass dieser ebenso wie die obere Führung 21 eine axiale Bewegung des Messbechers 11 zulässt und gleichzeitig verhindert, dass das Ausflussteil 15 den rotationssymmetrischen Körper 2 berühren kann.

Durch den vorgestellten Aufbau der Vorrichtung kann der Messbecher 11 bei eingesetztem Auffangbehälter 1 besonders vorteilhafter Weise eingesetzt und wieder ausgebaut werden. Ein Austausch des Bechers 11 ist damit sehr einfach und schnell realisierbar. Bevorzugt ist die Nase 25a oder die weitere Nase 25a des Klemmrings 23 so ausgestaltet, dass diese gleichzeitig als Griff dient, an dem der Messbecher 11 angehoben und aus dem Messwertgeber 4 herausgehoben werden kann.

Mit Hilfe einer Deckelplatte 26, dargestellt in Fig. 1, kann der Messwertgeber 4 nach oben hin abgedeckt werden. Diese Platte hat mehrere Funktionen. Zum einen sorgt sie dafür, dass der Klemmring 23 nicht aus der vorgesehenen Nut im rotationssymmetrischen Behälter 2 herausspringen kann, indem an der Deckelplatte angeformte Streben 28 von oben auf den Klemmring 23 drücken. Die notwendigen Andruckkräfte werden durch eine am flexiblen Auffangbehälter 1 angeformte umlaufende Lippendichtung 29 bereitgestellt, welche die Deckelplatte nach korrekter Installation nach unten drückt und gleichzeitig den Auffangbehälter 1 gegen die Deckelplatte 26 abdichtet.

Eine weitere Funktion der Deckelplatte 26 besteht darin, das Eindringen von Unrat in den Messwertgeber 4 zu verhindern. Dies wird erfindungsgemäß dadurch sichergestellt, dass der Harn durch Schlitze/Löcher (nicht dargestellt) in der Deckelplatte fließt.

Der gemäß der Beschreibung zu den Figuren 1 bis 4 aufgebaute Messwertgeber 4 ist von der erfindungsgemäßen Vorrichtung zur Wägemessung einer Harnströmung umfasst. Wie im einleitenden Teil der Beschreibung herausgestellt, stellen im Rahmen der Wägemessung insbesondere Impulsanteile ungewollte Störungen dar, die es folglich weitest möglich zu vermeiden gilt.

Im Rahmen der Erfindung wird dies dadurch gelöst, indem die Deckelplatte 26 mittels Einkerbungen 27 den gesammelten Harn möglichst zentral auf ein an die Deckelplatte angeformtes rotationssymmetrisches Prallelement 30 führt. Das Prallelement 30 dient dazu, die senkrechte Flüssigkeitsströmung umzuleiten und möglichst waagerecht gegen die Behälterwandung der Messkammer 13 umlaufend zu leiten und hiermit der Strömung ihre Geschwindigkeitskomponente in der gewollten Verschiebungs- bzw. Auslenkungsrichtung des Messbechers 11 zu nehmen.

Die Vorrichtung ist besonders einfach und wartungsfrei, da der Messwertgeber 4 in Abhängigkeit von der Batteriekapazität im Urinal verbleiben kann und nur der Messbecher 11 für einen regelmäßigen Austausch herausgenommen und gegen einen neuen Becher ausgewechselt wird. Ein permanenter Verbleib des Messbechers 11 im Messwertgeber 4 ist nicht wünschenswert, da eine Anlagerung von Urinstein und Schmutz nicht ausgeschlossen werden kann.

Die Vorrichtung ist weitestgehend gegen Vandalismus geschützt, indem der eigentliche Messwertgeber 4 verdeckt unterhalb vom Auffangbehälter 1 im Urinal angeordnet ist und von oben durch die Deckelplatte 26 verschlossen wird. Die Auswertungseinrichtung ist durch eine robuste Auf- oder Unterputzmontage unter einem geeigneten dauerfesten Material, beispielsweise Edelstahl, gegen Zerstörung geschützt. Das Display ist durch eine Platte gegen mechanische Zerstörung geschützt, wobei diese Platte gleichzeitig einen Blickschutz darstellt. Um die Interaktion mit der Auswertungseinrichtung zu ermöglichen, ist das berührungssensitive Element (touch panel) bevorzugt kapazitiv ausgelegt.

Die Vorrichtung ist kostengünstig, da das einzig bewegliche Teil des Sensorsystems (der Messbecher) günstig als Kunststoffspritzgussteil hergestellt werden kann, in den der oder die Magnete eingepresst werden. Während alle anderen Komponenten wieder verwendbar sind, ist der Messbecher in regelmäßigen Abständen zu tauschen. Der Austausch ist sehr einfach und lässt sich von einem Nichttechniker vornehmen, indem die Deckelplatte herausgenommen und der Messbecher nach oben herausgenommen wird. Der Einbau ist in umgekehrter Reihenfolge möglich.

Die Vorrichtung ist in bestehende Sanitäranlagen, insbesondere Urinale, nachrüstbar, indem der Sammelbehälter, auch Auffangbehälter genannt, urinalspezifisch angepasst werden kann.

Die Vorrichtung ist vollständig selbstbedienbar über eine Benutzerschnittstelle auf der Auswertungseinheit.

Die Vorrichtung kann sowohl im medizinischen wie auch im nichtmedizinischen Umfeld eingesetzt werden.

Der erfindungsgemäße Messwertgeber 4 ist, gegebenenfalls durch eine Variation des Auffangbehälters 1, auch zum Einsatz in Kombination mit einem handelsüblichen WC Becken geeignet.

## Patentansprüche

1. Vorrichtung zur Messung einer Harnströmung eines Patienten
- mit einem Messbecher (11), der eine Öffnung, die dem Abfluss von Urin dient, aufweist und gegenüber einem Messwertgeber (4) derart angeordnet ist, dass eine Verschiebung (dZ) des Messbechers (11) gegenüber dem Messwertgeber (4) als Maß für ein Volumen von in dem Messbecher (11) gesammeltem Urin detektierbar ist, wobei
- die Verschiebung (dZ) von einer Erhöhung eines Gesamtgewichtes des Messbechers (11) bewirkt ist, die beim Strömen des Urins durch den Messbecher (11) hindurch dem Gewicht des im Messbecher (11) gesammelten Urins entspricht, **dadurch gekennzeichnet, dass**
- an dem Messbecher (11) ein Ringmagnet oder eine Mehrzahl von Magneten (16) gleicher Polungsrichtung angebracht ist,
- an dem Messwertgeber (4) ein zweiter Ringmagnet oder eine Mehrzahl von zweiten Magneten (12) gleicher Polungsrichtung angebracht ist,
- die Magnetpole des Ringmagneten oder der Mehrzahl von Magneten (16) zu den Magnetpolen des zweiten Ringmagneten oder der Mehrzahl von zweiten Magneten (12) angeordnet sind, um eine abstoßende oder anziehende Magnetkraft zwischen dem Messbecher (11) und dem Messwertgeber (4) zu erzeugen, wobei
- eine Lage des Messbechers (11) gegenüber dem Messwertgeber (4) dadurch definiert ist, dass die Magnetkraft durch eine der Magnetkraft entgegengesetzte Gesamtgewichtskraft, die auf den Messbecher (4) wirkt, ausgeglichen ist.

2. Vorrichtung nach Anspruch 1, bei der der Messbecher (11) innerhalb des Messwertgebers (4) durch ein Führungsmittel (20-25b) derart geführt ist, dass eine Bewegung des Messbechers (11) auf eine im Wesentlichen translatorische Bewegung (11a) beschränkt ist.

3. Vorrichtung nach Anspruch 2, bei der das Führungsmittel (20-25b) Verbindungsstreben (24) umfasst, über die der Messbecher (11) und der Messwertgeber (4) miteinander verbunden sind.

4. Vorrichtung nach einem der vorhergehenden Ansprüche, bei der durch das Strömen des Urins durch den Messbecher (11) hindurch die Gesamtgewichtskraft gegenüber der Magnetkraft erhöht ist und die Lage des Messbechers (11) gegenüber dem Messwertgeber (4) in Richtung (Z) der Gesamtgewichtskraft verschoben ist.

5. Vorrichtung nach einem der vorherigen Ansprüche, bei der von dem Messwertgeber (4) die Verschiebung (dZ) des Messbechers (11) gegenüber dem Messwertgeber (4) und/oder eine Ableitung der Verschiebung (dZ) nach der Zeit mittels eines Sensorelementes (7), bevorzugt berührungslos, detektierbar sind/ist.

6. Vorrichtung nach einem der vorhergehenden Ansprüche, bei der die Verschiebung (dZ) des Messbechers (11) gegenüber dem Messwertgeber (4) und/oder eine Ableitung der Verschiebung (dZ) nach der Zeit in einen Flusswert umrechenbar sind/ist und der Flusswert von dem Messwertgeber (4) an eine Auswertungseinheit übertragbar ist.

7. Vorrichtung nach Anspruch 6, bei der die Auswertungseinheit zur Initialisierung der Messung einer Harnströmung, insbesondere über ein Touchpanel-Display, eingerichtet ist.

8. Vorrichtung nach einem der vorherigen Ansprüche, bei der der Messbecher (11) einen Strömungswiderstand (14) umfasst, der in dem Messbecher (11) derart angeordnet ist, dass der Urin der Gewichtskraft folgend durch den Strömungswiderstand (14) hindurch strömen kann und beim Strömen des Urins durch den Strömungswiderstand (14) hindurch der Urin gestaut werden kann zur Sammlung des Urins in dem Messbecher (11) .

9. Vorrichtung nach Anspruch 8, bei der der Strömungswiderstand (14) eine Querschnittsfläche (19a, 19b, 19c), durch die hindurch der Urin aus dem Messbecher (11) herausströmen kann, aufweist, die derart proportional ist zu einem Füllstand (18a, 18b, 18c) des Urins in dem Messbecher (11), dass der Strömungswiderstand mit höheren Harnströmung kleiner wird.

10. Vorrichtung nach Anspruch 9, bei der der Strömungswiderstand (14) in einer Draufsicht ein sternförmiges Profil aufweist und in einer Seitenansicht des Strömungswiderstandes die Grundform einem Kegelstumpf entspricht, wobei der Kegelstumpf durch zackenförmige Ausnehmungen (17) bis zum Grund einer Messkammer (13) des Messbechers (11) unterbrochen ist.

11. Vorrichtung nach einem der vorherigen Ansprüche, bei der ein Auffangbehälter (1) zum Sammeln des Urins vorgesehen ist, über den der gesammelte Urin in den Messwertgeber (4) geleitet werden kann.

12. Vorrichtung nach Anspruch 11, bei der zwischen dem Auffangbehälter (1) und dem Messwertgeber (4) ein den Messwertgeber (4) abdeckender Deckel (26) angeordnet ist, in den der von dem Auffangbehälter (1) gesammelte Urin geleitet werden kann.

13. Vorrichtung nach Anspruch 12, bei der der Deckel (26) derart geformt ist, dass beim Leiten des von dem Auffangbehälter (1) gesammelten Urins in den Deckel (26) der in Richtung (Z) der Gewichtskraft des Urins durch eine Öffnung oder mehrere Öffnungen (27) im Deckel vertikal (Z) abfließende Urin über ein Prallelement (30), das bevorzugt als Teil des Deckels (26) ausgebildet ist, in eine im Wesentlichen horizontale (X, Y) Strömungsrichtung umgeleitet ist.

14. Vorrichtung nach Anspruch 13, bei der das Prallelement (30) rotationssymmetrisch ausgebildet ist und einen sich vergrößernden Durchmesser in einer Richtung (Z) aufweist, die beim Abfließen des Urins der Richtung (Z) der Gewichtskraft des abfließenden Urins entspricht.

15. Verfahren zur Messung einer Harnströmung eines Patienten, bei dem
- ein Messbecher (11), der eine Öffnung, die dem Abfluss von Urin dient, aufweist und gegenüber einem Messwertgeber (4) derart angeordnet wird, dass eine Verschiebung (dZ) des Messbechers (11) gegenüber dem Messwertgeber (4) als Maß für ein Volumen von in dem Messbecher (11) gesammeltem Urin detektiert werden kann, wobei die Verschiebung (dZ) von einer Erhöhung eines Gesamtgewichtes des Messbechers (11) bewirkt wird, die beim Strömen des Urins durch den Messbecher (11) hindurch dem Gewicht des im Messbecher (11) gesammelten Urins entspricht,
- der Urin durch den Messbecher (11) hindurch strömt, und
- die Verschiebung (dZ) des Messbechers (11) gegenüber dem Messwertgeber (4) beim Strömen des Urins durch den Messbecher (11) hindurch als Maß für das Volumen des im Messbecher (11) gesammelten Urins detektiert wird,
**dadurch gekennzeichnet, dass**
- an dem Messbecher (11) ein Ringmagnet oder eine Mehrzahl von Magneten (16) gleicher Polungsrichtung angebracht wird,
- an dem Messwertgeber (4) ein zweiter Ringmagnet oder eine Mehrzahl von zweiten Magneten (12) gleicher Polungsrichtung angebracht wird,
- die Magnetpole des Ringmagneten oder der Mehrzahl von Magneten (16) zu den Magnetpolen des zweiten Ringmagneten oder der Mehrzahl von zweiten Magneten (12) angeordnet werden, um eine abstoßende oder anziehende Magnetkraft zwischen dem Messbecher (11) und dem Messwertgeber (4) zu erzeugen, wobei
- eine Lage des Messbechers (11) gegenüber dem Messwertgeber (4) dadurch definiert wird, dass die Magnetkraft durch eine der Magnetkraft entgegengesetzte Gesamtgewichtskraft, die auf den Messbecher (4) wirkt, ausgeglichen wird.

## Claims

1. A device for measuring a urine flow of a patient
- with a measurement beaker (11) which has an opening for draining the urine and which is arranged with respect to a measuring transducer (4) in such a manner that a displacement (dZ) of the measurement beaker (11) with respect to the measuring transducer (4) is detectable as measurement for a volume of urine collected in the measurement beaker (11), wherein
- the displacement (dZ) is produced by an increase of a total weight of the measurement beaker (11), which, when the urine flows through the measurement beaker (11), corresponds to the weight of the urine collected in the measurement beaker (11),
**characterised in that**,
- on the measurement beaker (11), a ring magnet or a plurality of magnets (16) of identical polarization direction is attached,
- on the measuring transducer (4), a second ring magnet or a plurality of second magnets (12) of identical polarization direction is attached,
- the magnetic poles of the ring magnet or of the plurality of ring magnets (16) are arranged relative to the magnetic poles of the second ring magnet or the plurality of second magnets (12) so as to generate a repelling or an attractive magnetic force between the measurement beaker (11) and the measuring transducer (4), wherein
- a position of the measurement beaker (11) with respect to the measuring transducer (4) is defined **in that** the magnetic force is balanced by a total weight force countering the magnetic force and acting on the measurement beaker (4).

2. The device according to Claim 1, in which the measurement beaker (11) is guided within the measuring transducer (4) by a guide means (20-25b) in such a manner that a movement of the measurement beaker (11) is limited to a substantially translational movement (11a).

3. The device according to Claim 2, in which the guide means (20-25b) comprises connecting struts (24), via which the measurement beaker (11) and the measuring transducer (4) are connected to one another.

4. The device according to any one of the preceding claims, in which, due to the flow of the urine through the measurement beaker (11), the total weight force with respect to the magnetic force is increased, and the position of the measurement beaker (11) is displaced with respect to the measuring transducer (4) in direction (Z) of the total weight force.

5. The device according to any one of the preceding claims, in which the displacement (dZ) of the measurement beaker (11) with respect to the measuring transducer (4) and/or a derivative of the displacement (dZ) with respect to time is/are detectable by the measuring transducer (4) by means of a sensor element (7), preferably in a contactless manner.

6. The device according to any one of the preceding claims, in which the displacement (dZ) of the measurement beaker (11) with respect to the measuring transducer (4) and/or a derivative of the displacement (dZ) with respect to time is/are convertible into a flow value, and the flow value is transferable from the measuring transducer (4) to an evaluation unit.

7. The device according to Claim 6, in which the evaluation unit is set up for initializing the measurement of a urine flow, in particular via a touch panel display.

8. The device according to any one of the preceding claims, in which the measurement beaker (11) comprises a flow resistance (14) which is arranged in the measurement beaker (11) in such a manner that the urine, as a result of weight force, can flow through the flow resistance (14) and, when the urine flows through the flow resistance (14), the urine can accumulate in the measurement beaker (11) for the collection of the urine.

9. The device according to Claim 8, in which the flow resistance (14) comprises a cross-sectional area (19a, 19b, 19c) through which the urine can flow out of the measurement beaker (11), which is proportional to a filling level (18a, 18b, 18c) of the urine in the measurement beaker (11), in such a manner that the flow resistance decreases with increasing urine flow.

10. The device according Claim 9, in which the flow resistance (14) has a star-shaped profile in a top view and, in a side view of the flow resistance, the basic shape corresponds to a truncated cone, wherein the truncated cone is interrupted by tooth-shaped recesses (17) down to the bottom of a measurement chamber (13) of the measurement beaker (11).

11. The device according to any one of the preceding claims, in which a collection container (1) for collecting the urine is provided, via which the collected urine can be guided into the measuring transducer (4).

12. The device according to Claim 11, in which, between the collection container (1) and the measuring transducer (4), a cover (26) covering the measuring transducer (4) is arranged, into which the urine collected by the collection container (1) can be led.

13. The device according to Claim 12, in which the cover (26) is shaped in such a manner that, when the urine collected by the collection container (1) is led into the cover (26), the urine draining vertically (Z) in direction (Z) of the weight of the urine through an opening or several openings (27) in the cover is deflected via an impact element (30) which is preferably formed as a portion of the cover (26), in a substantially horizontal (X, Y) flow direction.

14. The device according to Claim 13, in which the impact element (30) is of rotationally symmetric design and has a diameter which increases in a direction (Z) which corresponds to the weight force of the draining urine, when the urine drains in the direction (Z).

15. A method for measuring a urine flow of a patient, in which
- a measurement beaker (11) which has an opening for draining urine and is arranged with respect to a measuring transducer (4) in such a manner that a displacement (dZ) of the measurement beaker (11) with respect to the measuring transducer (4) can be detected as measurement for a volume of urine collected in the measurement beaker (11), wherein the displacement (dZ) is produced by an increase in the total weight of the measurement beaker (11), which, when the urine flows through the measurement beaker (11), corresponds to the weight of the urine collected in the measurement beaker (11),
- the urine flows through the measurement beaker (11), and,
- when the urine flows through the measurement beaker (11), the displacement (dZ) of the measurement beaker (11) with respect to the measuring transducer (4) is detected as measurement for the volume of the urine collected in the measurement beaker (11),
**characterised in that**,
- on the measurement beaker (11), a ring magnet or a plurality of magnets (16) of identical polarization direction is attached,
- on the measuring transducer (4), a second ring magnet or a plurality of second magnets (12) of identical polarization direction is attached,
- the magnetic poles of the ring magnets or of the plurality of magnets (16) are arranged with respect to the magnetic poles of the second ring magnets or of the plurality of second magnets (12) so as to generate a repelling or an attractive magnetic force between the measurement beaker (11) and the measuring transducer (4), wherein
- a position of the measurement beaker (11) with respect to the measuring transducer (4) is defined **in that** the magnetic force is balanced by a total weight force countering the magnetic force and acting on the measurement beaker (4).

## Revendications

1. Dispositif destiné à mesurer un débit urinaire d'un patient
- avec un gobelet doseur (11), qui présente une ouverture qui sert à la sortie de l'urine et est disposé par rapport à un capteur (4) de telle sorte qu'un décalage (dZ) du gobelet doseur (11) par rapport au capteur (4) puisse être détecté en tant que mesure d'un volume de l'urine collectée dans le gobelet doseur (11), dans lequel
- le décalage (dZ) est entraîné par une augmentation d'un poids total du gobelet doseur (11) qui correspond lors de l'écoulement de l'urine dans le gobelet doseur (11) au poids de l'urine collectée dans le gobelet doseur (11), **caractérisé en ce que**
- sur le gobelet doseur (11) est monté(e) un aimant annulaire ou une pluralité d'aimants (16) de même polarité,
- sur le capteur (4) est monté(e) un deuxième aimant annulaire ou une pluralité de deuxièmes aimants (12) de même polarité,
- les pôles magnétiques de l'aimant annulaire ou de la pluralité d'aimants (16) sont disposés vers les pôles magnétiques du deuxième aimant annulaire ou de la pluralité de deuxièmes aimants (12) pour générer une force magnétique répulsive ou attractive entre le gobelet doseur (11) et le capteur (4), dans lequel
- une position du gobelet doseur (11) par rapport au capteur (4) est définie de telle sorte que la force magnétique soit compensée par une force de gravité totale opposée à la force magnétique, qui agit sur le gobelet doseur (4).

2. Dispositif selon la revendication 1, dans lequel le gobelet doseur (11) est guidé à l'intérieur du capteur (4) par un moyen de guidage (20-25b) de telle sorte qu'un mouvement du gobelet doseur (11) soit limité à un mouvement (11a) sensiblement de translation.

3. Dispositif selon la revendication 2, dans lequel le moyen de guidage (20-25b) comprend des barres de liaison (24) par le biais desquelles le gobelet doseur (11) et le capteur (4) sont reliés l'un à l'autre.

4. Dispositif selon l'une des revendications précédentes, dans lequel l'écoulement de l'urine dans le gobelet doseur (11) entraîne l'augmentation de la force de gravité totale par rapport à la force magnétique et la position du gobelet doseur (11) par rapport au capteur (4) est décalée dans la direction (Z) de la force de gravité totale.

5. Dispositif selon l'une des revendications précédentes, dans lequel le décalage (dZ) du gobelet doseur (11) par rapport au capteur (4) et/ou une déduction du décalage (dZ) selon le temps peut/peuvent être détecté(s) par le capteur (4) au moyen d'un élément détecteur (7), de préférence sans contact.

6. Dispositif selon l'une des revendications précédentes, dans lequel le décalage (dZ) du gobelet doseur (11) par rapport au capteur (4) et/ou une déduction du décalage (dZ) selon le temps peut/peuvent être calculé (s) dans une valeur de flux et la valeur de flux peut être transmise du capteur (4) à une unité d'analyse.

7. Dispositif selon la revendication 6, dans lequel l'unité d'analyse est constituée pour l'initialisation de la mesure d'un débit urinaire, en particulier par le biais d'un écran tactile.

8. Dispositif selon l'une des revendications précédentes, dans lequel le gobelet doseur (11) comprend une résistance à l'écoulement (14) qui est disposée dans le gobelet doseur (11) de telle sorte que l'urine puisse s'écouler en suivant la force de gravité au travers de la résistance à l'écoulement (14) et lors de l'écoulement de l'urine au travers de la résistance à l'écoulement (14), l'urine peut être retenue pour la collecte de l'urine dans le gobelet doseur (11).

9. Dispositif selon la revendication 8, dans lequel la résistance à l'écoulement (14) présente une section transversale (19a, 19b, 19c) au travers de laquelle l'urine venant du gobelet doseur (11) peut sortir, qui est proportionnelle à un niveau de remplissage (18a, 18b, 18c) de l'urine dans le gobelet doseur (11) de telle sorte que la résistance à l'écoulement soit inférieure lorsque le débit urinaire augmente.

10. Dispositif selon la revendication 9, dans lequel la résistance à l'écoulement (14) présente, sur une vue du dessus, un profil en forme d'étoile et sur une vue latérale de la résistance à l'écoulement, la forme de base correspond à celle d'un cône tronqué, dans lequel le cône tronqué est interrompu par des évidements en forme de dents (17) jusqu'au fond d'une chambre de mesure (13) du gobelet doseur (11).

11. Dispositif selon l'une des revendications précédentes, dans lequel un récipient collecteur (1) est prévu pour la collecte de l'urine, par le biais duquel l'urine collectée peut être conduite dans le capteur (4).

12. Dispositif selon la revendication 11, dans lequel entre le récipient collecteur (1) et le capteur (4) est disposé un couvercle (26) recouvrant le capteur (4), dans lequel l'urine collectée par le récipient collecteur (1) peut être conduite.

13. Dispositif selon la revendication 12, dans lequel le couvercle (26) est formé de telle sorte que lors de la conduite de l'urine collectée par le récipient collecteur (1) dans le couvercle (26), l'urine sortant verticalement (Z) dans la direction (Z) de la force de gravité de l'urine soit dérivée, par une ouverture ou plusieurs ouvertures (27) dans le couvercle sur un élément d'impact (30) qui est conçu de préférence comme une partie du couvercle (26) dans une direction d'écoulement sensiblement horizontale (X, Y).

14. Dispositif selon la revendication 13, dans lequel l'élément d'impact (30) est conçu de façon symétrique en rotation et présente un diamètre s'agrandissant dans la direction (Z), qui correspond lors de la sortie de l'urine, à la direction (Z) de la force de gravité de l'urine sortant.

15. Procédé de mesure d'un débit urinaire d'un patient, dans lequel
- un gobelet doseur (11) présente une ouverture qui sert à la sortie de l'urine et est disposé par rapport à un capteur (4) de telle sorte qu'un décalage (dZ) du gobelet doseur (11) par rapport au capteur (4) puisse être détecté en tant que mesure d'un volume de l'urine collectée dans le gobelet doseur (11), dans lequel le décalage (dZ) est entraîné par une augmentation d'un poids total du gobelet doseur (11) qui correspond lors de l'écoulement de l'urine dans le gobelet doseur (11) au poids de l'urine collectée dans le gobelet doseur (11),
- l'urine s'écoule dans le gobelet doseur (11), et
- le décalage (dZ) du gobelet doseur (11) par rapport au capteur (4) lors de l'écoulement de l'urine dans le gobelet doseur (11) est détecté en tant que mesure du volume de l'urine collectée dans le gobelet doseur (11),
**caractérisé en ce que**
- sur le gobelet doseur (11) est monté(e) un aimant annulaire ou une pluralité d'aimants (16) de même polarité,
- sur le capteur (4) est monté(e) un deuxième aimant annulaire ou une pluralité de deuxièmes aimants (12) de même polarité,
- les pôles magnétiques de l'aimant annulaire ou de la pluralité d'aimants (16) sont disposés vers les pôles magnétiques du deuxième aimant annulaire ou de la pluralité de deuxièmes aimants (12) pour générer une force magnétique répulsive ou attractive entre le gobelet doseur (11) et le capteur (4), dans lequel
- une position du gobelet doseur (11) par rapport au capteur (4) est définie de telle sorte que la force magnétique soit compensée par une force de gravité totale opposée à la force magnétique, qui agit sur le gobelet doseur (4).
